# EUROPÄISCHE PATENTSCHRIFT

(11) **EP 0 579 617 B1**
(45) Veröffentlichungstag und Bekanntmachung des Hinweises auf die Patenterteilung: **04.09.1996**
(21) Anmeldenummer: 92906255.2
(22) Anmeldetag: 04.03.1992
(51) Int. Cl.: C07C 51/235

(54) **VERFAHREN ZUR HERSTELLUNG VON 3-HYDROXYPROPIONSÄURE**
PROCESS FOR PREPARING 3-HYDROXYPROPIONIC ACID
PROCEDE DE PREPARATION D'ACIDE 3-HYDROXYPROPIONIQUE

(30) Priorität: 13.03.1991 DE 4107987
(43) Veröffentlichungstag der Anmeldung: 26.01.1994
(73) Patentinhaber: Henkel Kommanditgesellschaft auf Aktien, 40191 Düsseldorf (DE)
(72) Erfinder: BEHR, Arno, D-4000 Düsseldorf 13 (DE); BOTULINSKI, Andreas, D-4047 Dormagen 5 (DE); CARDUCK, Franz-Josef, D-5657 Haan (DE); SCHNEIDER, Michael, D-5620 Verbert 11 (DE)
(86) Internationale Anmeldenummer: EP9200477
(87) Internationale Veröffentlichungsnummer: WO9216489

(56) Entgegenhaltungen:
- DE-B- 1 035 639
- US-A- 3 897 489
- CHEMICAL ABSTRACTS, vol. 86, no. 13, 28. März 1977, Columbus, Ohio, US; abstract no. 89185K, KIYOURA,TADAMITSU: 'ALKALI GLYCOLATES & JP-A-76 86415, PUBLISHED 29-07-1976' Seite 487 ;Spalte 1 ;

## Beschreibung

Die Erfindung betrifft ein Verfahren zur Herstellung von Alkalisalzen der 3-Hydroxypropionsäure durch Oxidation von 1,3-Propandiol in wäßrig-alkalischer Lösung in Gegenwart eines Palladiumkatalysators.

3-Hydroxypropionsäure bzw. deren Salze sind wertvolle Synthesebausteine der organischen Synthese. Üblicherweise wird 3-Hydroxypropionsäure durch Wasseranlagerung an Acrylsäure oder durch Umsetzung von Ethylenchlorhydrin mit Natriumcyanid und nachfolgender Hydrolyse des gebildeten β-Propiolactons hergestellt [Ullmann's Encyclopedia of Industrial Chemistry, 5.Auflage, Band A-13, S. 507-517]. In beiden Fällen handelt es sich um Verfahren, in denen toxische Substanzen gehandhabt werden müssen. Es wurde daher nach einem Verfahren gesucht, um das toxikologisch unbedenkliche 1,3-Propandiol, das durch Fermentation aus Glycerin leicht und in hoher Ausbeute zugängliche ist, oxidativ in 3-Hydroxypropionsäure bzw. dessen Alkalisalze zu überführen.

Die Oxidation von 1,3-Propandiol, fortan kurz als Diol bezeichnet, in Gegenwart eines Edelmetallkatalysators ist literaturbekannt. So beansprucht die japanische Offenlegungsschrift JP 56/5433 der Firma Sanyo ein Verfahren zur Herstellung von Malonsäure durch Umsetzung von 1,3-Propandiol mit Sauerstoff oder einem sauerstoffhaltigen Gas. Vorzugsweise wird das Verfahren in Gegenwart eines Platingruppenkatalysators durchgeführt. Gemäß der Sanyo-Anmeldung ist bei Einsatz von 3,3 Gew.-% Palladium bezogen auf das Diol Malonsäure in hohen Ausbeuten zugänglich.

Die Aufgabe der vorliegenden Erfindung bestand darin, ein Verfahren zu entwickeln, bei dem 1,3-Propandiol mit guter Ausbeute zu 3-Hydroxypropionsäure oxidiert wird.

Diese Aufgabe wurde erfindungsgemäß gelöst durch Verfahren zur Herstellung eines Alkalimetallsalzes von 3-Hydroxypropionsäure durch Umsetzung von 1,3-Propandiol mit Sauerstoff oder einem sauerstoffhaltigen Gas in wäßrigalkalischer Lösung in Gegenwart eines Palladium enthaltenden Trägerkatalysators , dadurch gekennzeichnet, daß man
a) den Katalysator in einer Menge einsetzt, die 0,1 bis 3,0 Gew.-% Palladium - bezogen auf 1,3-Propandiol - entspricht, und
b) die Umsetzung bei einer Temperatur im Bereich von 40 bis 55°C durchführt. Als Katalysator wird Palladium auf einem festen Träger, z.B. Aktivkohle oder Aluminiumoxid eingesetzt, wobei die Menge des im Katalysator enthaltenen Palladiums 0,5 bis 10 Gew.-%, vorzugsweise 2 bis 5 Gew.-% bezogen auf den Träger beträgt.

Die Ursache für die Funktionsfähigkeit des erfindungsgemäßen Verfahrens ist darin zu sehen, daß die überwiegende Bildung von 3-Hydroxypropionsäure (selektive Oxidation von 1,3-Propandiol) gegenüber der konkurrierenden Bildung von Malonsäure (vollständige Oxidation von 1,3-Propandiol) bei Verwendung der erfindungsgemäß niedrigen Katalysatorkonzentration - bezogen auf das Diol - bevorzugt ist.

In einer bevorzugten Ausführungsform der Erfindung beträgt die Menge des im Katalysator enthaltenen Palladiums - bezogen auf 1,3-Propandiol - 0,1 bis 1,0 Gew.-%.

Es hat sich gezeigt, daß gute Ausbeuten an 3-Hydroxypropionsäure auch dann erhalten werden, wenn der Katalysator - über das vorhandene Palladium hinaus - zusätzlich Platin und/oder Wismut enthält. Dabei soll die Gesamtmenge an Platin und/oder Wismut höchstens das Doppelte der Gewichtsmenge an Palladium betragen. Besonders gute Ergebnisse wurden mit einem Katalysator erzielt, der 4 Gew.-% Palladium, 1 Gew.-% Platin und 5 Gew.-% Wismut auf gepulverter Aktivkohle enthielt.

Der Katalysator wird vor dem Einsatz üblicherweise aktiviert. Dies geschieht in einfacher Weise dadurch, daß er in Wasser dispergiert und anschließend zur Verdrängung von anhaftendem Sauerstoff mit inerten Gasen, z.B mit Wasserstoff und/oder Stickstoff in Kontakt gebracht wird.

Der Katalysator kann im Zuge des erfindungsgemäßen Verfahrens mehrfach wiederverwendet werden, ohne daß Einbußen in bezug auf die Ausbeute hingenommen werden müssen. Es wurde im Gegenteil sogar beobachtet, daß der Katalysator erst dann seine volle Aktivität entfaltet, wenn er mindestens 1- bis 3-mal wiederverwendet worden ist.

Die Konzentration des Diols in der Reaktionsmischung unterliegt keinen besonderen Einschränkungen, jedoch werden 5 bis 20 Gew.-% bevorzugt. Dabei ist es von besonderem Vorteil, die Konzentration des Diols in der Reaktionsmischung auf Werte im Bereich von 6 bis 12 Gew.-%, insbesondere von 8 bis 10 Gew.-% einzustellen; in diesem Fall läßt sich - bei weiter hoher Ausbeute - die Menge des im Katalysator enthaltenen Palladiums weiter auf 0,1 bis 0,3 Gew.-% reduzieren.

Die Oxidation von 1,3-Propandiol wird erfindungsgemäß im alkalischen Milieu durchgeführt. Dadurch wird erreicht, daß die gebildete 3-Hydroxypropionsäure neutralisiert und auf diese Weise einem partiellen, ausbeutemindernden Abbau durch Decarboxylierung entzogen wird. Der pH-Wert der wäßrig alkalischen Reaktionsgemische soll 8 bis 13, vorzugsweise 9 bis 12 betragen. Besonders gute Ergebnisse werden bei pH-Werten von 10 bis 11 erzielt.

Die Oxidation des Diols wird derart durchgeführt, daß der pH-Wert der Reaktionsmischung während der gesamten Reaktion konstant gehalten wird. Diese pH-Konstanz kann z.B. durch Kopplung eines pH-Meters, das den pH-Wert der Reaktionsmischung kontinuierlich überwacht, mit einer Dosiervorrichtung, die das entsprechende Alkalihydroxid enthält, gesteuert werden. Als Alkalihydroxide werden dabei vorzugsweise Natriumhydroxid und Kaliumhydroxid, insbesondere in Form ihrer wäßrigen Lösungen eingesetzt. Die Konzentration des verwendeten wäßrigen Alkalihydroxids unterliegt keiner besonderen Beschränkung, es ist jedoch klar, daß die Verwendung sehr verdünnter Lösungen im Hinblick auf eine optimale Raumausnutzung des Reaktors unökonomisch ist. Man wird daher aus praktischen Erwägungen 20 bis 50 gewichtsprozentige wäßrige Alkalihydroxide verwenden. Im Falle von NaOH hat sich die Verwendung einer 30 gew.%-igen, d.h. 10-normalen Lösung als praktisch erwiesen.

Die Oxidation des Diols wird im erfindungsgemäßen Verfahren vorzugsweise bei Temperaturen von 40 bis 55 °C durchgeführt. Höhere Temperaturen ergeben keine signifikanten Ausbeutesteigerungen und bergen allenfalls die Gefahr einer Dehydratisierung der 3-Hydroxypropionsäure zu Acrylsäure.

Im Zuge des erfindungsgemäßen Verfahrens wird das Reaktionsgemisch mit Sauerstoff oder dem sauerstoffhaltigen Gas, z.B. Luft, in Kontakt gebracht. Dies ist z.B. in einfacher Weise dadurch möglich, daß die Luft unter Rühren in die Reaktionsmischung eingeblasen wird. Es hat sich gezeigt, daß dabei eine Flußrate von 30 Normlitern Luft pro Stunde - bezogen auf 700 bis 1000 ml der Reaktionsmischung - , besonders geeignet ist. Bei geringeren Flußraten, z.B. 10 Normlitern Luft pro Stunde, wird die Reaktionszeit erheblich verlängert und es kann zu einer unerwünschten Gelbfärbung der Reaktionsmischung kommen, bei höheren Flußraten ist der Katalysatoraustrag aus dem Reaktor zu hoch.

Die Reaktion kann bei Drucken von 1,0 bis 1,5 bar durchgeführt werden, vorzugsweise arbeitet man bei einem leichten Überdruck im Bereich von 1,01 bis 1,06 bar.

In kinetischen Untersuchungen wurde gefunden, daß der Verbrauch an Alkalihydroxid in dem erfindungsgemäßen Verfahren zeitabhängig ist: Der Alkaliverbrauch nimmt zunächst mit der Zeit linear zu und erreicht mit dem Ende der Reaktion einen Plateauwert. Aufgrund dieser Beobachtung kann daher der Endpunkt der Reaktion in einfacher Weise dadurch festgestellt werden, daß zur Konstanthaltung des pH-Wertes kein weiteres Alkalihydroxid mehr benötigt wird. An dieser Stelle wird die Reaktion abgebrochen und die Reaktionsmischung in üblicher Weise, beginnend mit dem Abfiltrieren des Katalysators aufgearbeitet.

Das erhaltene Alkalisalz der 3-Hydroxypropionsäure kann entweder direkt oder nach weiterem Aufkonzentrieren als wäßrige Lösung verwendet werden. Gewünschtenfalls kann das Alkalisalz durch Ansäuern, z.B. mittels eines sauren Ionenaustauschers, in die freie 3-Hydroxypropionsäure überführt werden, die gewünschtenfalls destillativ gereinigt werden kann.

Die folgenden Beispiele sollen den Gegenstand der Erfindung näher erläutern.

### Beispiele

### 1. Reagentien

Folgende Katalysatoren wurden verwendet:
a) 5 Gew.-% Pd auf Aktivkohle, mit einem Wassergehalt von 52,5 Gew.-%, unter der Bezeichnung Escat 10 kommerziell erhältlich (Fa. Engelhard).
b) 4 Gew.-% Pd, 1 Gew.-% Pt sowie 5 Gew.-% Bi auf Aktivkohle, mit einem Wassergehalt von 59,3 Gew.-%, unter der Bezeichnung Cef 196 raw kommerziell erhältlich (Fa. Degussa).

### 2. Versuchsapparatur

Die Oxidationen wurden in einem 2-Liter Druckautoklaven mit Turbinenrührer [1400 Umdrehungen pro Minute] durchgeführt. Luft und 30 gew.%-ige Natronlauge wurden von unten in die Reaktionsmischung eingeleitet. NaOH wurde kontinuierlich derart zudosiert, daß der pH-Wert der Mischung konstant blieb. Die Steuerung erfolgte über ein mit einem Widerstandthermometer (Pt-100) ausgerüsteten Dosimeter (Dulcometer, Fa. Prominent). Das Abgas gelangte durch einen Kühler (Kondensatabscheidung), einen Pufferbehälter, eine mit Wasser gefüllte Waschflasche, einen Trockenturn, ein Durchflußmeßgerät und einen Sauerstoff-Analysator (Servomex 570, Fa. Bühler). Bedingt durch Luftdruckschwankungen wurde das Gerät vor jedem Versuch neu geeicht. Die Sauerstoffzehrung wurde zeitabhängig durch einen angeschlossenen Schreiber kontinuierlich registriert. Der Luftdurchsatz wurde mittels eines Feinregulierventils auf einen Wert von 30 Normliter pro Stunde eingestellt. Der Reaktorinnendruck betrug 1,06 bar.

### 3. Versuchsbeschreibungen

Die angegebenen Katalysatormengen beziehen sich in allen Beispielen bzw. Vergleichsbeispielen auf die Trockensubstanz.

### Beispiel 1 (B1)

2,13g Katalysator (Escat 10) wurden in 300 ml Wasser dispergiert und über Nacht unter Wasserstoff aktiviert, wobei eine Menge von ca. 350 ml aufgenommen wurde. Der vorbereitete Katalysator wurde zusammen mit 77,6g (1 mol) 1,3-Propandiol und weiteren 500 ml Wasser in den Autoklaven überführt. Der Autoklav wurde verschlossen und die Reaktionsmischung unter einem Stickstoffstrom auf 50 °C erwärmt.
Die Reaktion wurde durch gleichzeitiges Zudosieren von Natronlauge und Luft gestartet. NaOH wurde kontinuierlich derart zudosiert, daß der pH-Wert konstant bei 11 lag. Der Luftdurchsatz betrug 30 Normliter pro Stunde. Sobald ein konstanter Wert der Natronlaugedosierung sowie eine damit verbundene konstante Sauerstoffrestzehrung erreicht war, wurde die Reaktion gestoppt, das Reaktionsgemisch durch Ausblasen der NaOH-Dosierungsleitung abgelassen und die Auswaage bestimmt. Nach dem Erkalten der Mischung wurde der Katalysator mit einer Nutsche abfiltriert und die Proben mittels HPLC (Shodex Ionpak C-811: Kationaustauschphase, 0,1 gewichtsprozentige wäßrige Phosphorsäure als Eluent, Ri-Detektion) analysiert. Die Ausbeuten an 3-Hydroxypropionsäure [in % der Theorie] sind zusammen mit weiteren Daten in Zeile 1 der Tabelle 1 zusammengestellt.

### Beispiele 2 bis 4 (B2 bis B4)

Versuch 1 wurde mit veränderten Katalysatormengen wiederholt. Die Ergebnisse sind in Tabelle 1 zusammangestellt. Es zeigt sich, daß optimale Ausbeuten an 3-Hydroxypropionsäure insbesondere bei niedrigen Katalysatorkonzentrationen erhalten werden.

### Vergleichsbeispiel 1 (V1)

Versuch 1 wurde mit einer deutlich höheren Menge an Katalysator wiederholt. Einzelheiten sind in Tabelle 1 zusammengestellt. Daraus wird deutlich, daß die Erhöhung der Katalysatormenge mit einer drastischen Ausbeuteverminderung an 3-Hydroxypropionsäure einhergeht.

### Beispiel 5 (B5)

13,1g Katalysator (Escat 10) wurden in 300 ml Wasser dispergiert und über Nacht unter Wasserstoff aktiviert, wobei eine Menge von ca. 900 ml aufgenommen wurde. Der vorbereitete Katalysator wurde zusammen mit 23,3g (300 mmol) 1,3-Propandiol und weiteren 400 ml Wasser in den Autoklaven überführt. Der Autoklav wurde verschlossen und die Reaktionsmischung unter einem Stickstoffstrom auf 40 °C erwärmt. Die weitere Versuchsdurchführung entsprach der des Beispiels 1. Die Ausbeute an 3-Hydroxypropionsäure betrug 65,6%, vergl. Tabelle 1.

### Vergleichsbeispiel 2 (V2)

Beispiel 5 wurde bei 60 °C wiederholt. Die Ausbeute an 3-Hydroxypropionsäure betrug 29%, vergl. Tabelle 1.

**Tabelle 1**

| Bsp. | 1,3-Propandiol | | Wasser [g] | Kata^{a)} [g] | Pd^{b)} [%] | Temp. [°C] | Zeit^{c)} [min] | Ausbeute^{d)} [%] |
|---|---|---|---|---|---|---|---|---|
| | [g] | [mmol] | | | | | | |
| B1 | 77,6 | 1000 | 800 | 2,13 | 0,14 | 50 | 1075^{e)} | 70,1 |
| B2 | 77,6 | 1000 | 800 | 3,80 | 0,24 | 50 | 490 | 74,7 |
| B3 | 77,6 | 1000 | 800 | 5,32 | 0,34 | 50 | 415 | 68,4 |
| B4 | 77,6 | 1000 | 800 | 10,64 | 0,69 | 50 | 330 | 51,5 |
| V1 | 77,6 | 1000 | 800 | 21,28 | 1,37 | 50 | 580 | 23,5 |
| B5 | 23,3 | 300 | 700 | 13,10 | 2,81 | 40 | 190 | 65,6 |
| V2 | 23,3 | 300 | 700 | 13,10 | 2,81 | 60 | 220 | 29,0 |

| | | | | | | | | |
|---|---|---|---|---|---|---|---|---|
| (a) Escat 10; die aufgeführten Katalysatormengen sind Trockeneinwaagen | | | | | | | | |
| (b) Gew.-% Palladium bezogen auf 1,3-Propandiol | | | | | | | | |
| (c) Reaktionszeit in Minuten | | | | | | | | |
| (d) Ausbeute an 3-Hydroxypropionsäure in Prozent der Theorie | | | | | | | | |
| (e) Luftdurchsatz: 10 Normliter pro Stunde | | | | | | | | |

### Beispiel 6 (B6)

13,1g Katalysator (Cef 196 raw) wurden in 300 ml Wasser dispergiert und über Nacht unter Wasserstoff aktiviert, wobei eine Menge von ca. 900 ml aufgenommen wurde. Der vorbereitete Katalysator wurde zusammen mit 31,1g (400 mmol) 1,3-Propandiol und weiteren 631 ml Wasser in den Autoklaven überführt. Der Autoklav wurde verschlossen und die Reaktionsmischung unter einem Stickstoffstrom auf 50 °C erwärmt. Die weitere Versuchsdurchführung entsprach der des Beispiels 1. Die Ausbeute an 3-Hydroxypropionsäure betrug 81,8 % vergl. Tabelle 2.

### Beispiele 7 bis 10 (B7 bis B10)

Versuch 6 wurde mit veränderten Konzentrationen von 1,3-Propandiol in der Reaktionsmischung - und damit indirekt auch mit veränderten Verhältnissen von Katalysator zu Diol - wiederholt. Details sowie die Ausbeuten an 3-Hydroxypropionsäure sind in Tabelle 2 zusammengestellt.

**Tabelle 2**

| Bsp. | 1,3-Propandiol | | Wasser [g] | Kata^{a)} [g] | Pd^{b)} [%] | Met^{c)} [%] | Temp. [°C] | Zeit^{d)} [min] | Ausbeute^{e)} [%] |
|---|---|---|---|---|---|---|---|---|---|
| | [g] | [mmol] | | | | | | | |
| B6 | 31,1 | 400 | 931 | 17,46 | 2,2 | 3,4 | 50 | 140 | 81,8 |
| B7 | 23,3 | 300 | 700 | 13,10 | 2,3 | 3,3 | 50 | 140 | 73,8 |
| B8 | 46,6 | 600 | 700 | 13,10 | 1,1 | 1,7 | 50 | 240 | 77,2 |
| B9 | 69,8 | 900 | 700 | 13,10 | 0,8 | 1,1 | 50 | 300 | 78,2 |
| B10 | 93,1 | 1200 | 700 | 13,10 | 0,6 | 0,8 | 50 | 570 | 70,5 |

| | | | | | | | | | |
|---|---|---|---|---|---|---|---|---|---|
| (a) Cef 196 raw; die aufgeführten Katalysatormengen sind Trockeneinwaagen | | | | | | | | | |
| (b) Gew.-% Palladium bezogen auf 1,3-Propandiol | | | | | | | | | |
| (c) Gew.-% (Platin + Wismut) bezogen auf 1,3-Propandiol | | | | | | | | | |
| (d) Reaktionszeit in Minuten | | | | | | | | | |
| (e) Ausbeute an 3-Hydroxypropionsäure in Prozent der Theorie | | | | | | | | | |

## Patentansprüche

1. Verfahren zur Herstellung eines Alkalimetallsalzes von 3-Hydroxypropionsäure durch Umsetzung von 1,3-Propandiol mit Sauerstoff oder einem sauerstoffhaltigen Gas in wäßrig-alkalischer Lösung in Gegenwart eines Palladium enthaltenden Trägerkatalysators , dadurch gekennzeichnet, daß man
a) den Katalysator in einer Menge einsetzt, die 0,1 bis 3,0 Gew.-% Palladium - bezogen auf 1,3-Propandiol - entspricht und
b) die Umsetzung bei einer Temperatur im Bereich von 40 bis 55 °C durchführt.

2. Verfahren gemäß Anspruch 1, dadurch gekennzeichnet, daß man den Katalysator in einer Menge einsetzt, die 0,1 bis 1,0 Gew.-% Palladium - bezogen auf 1,3-Propandiol - entspricht.

3. Verfahren gemäß Anspruch 2, dadurch gekennzeichnet, daß man 1,3-Propandiol in einer Menge einsetzt, die 6 bis 12 Gew.-% - bezogen auf das Reaktionsgemisch - entspricht.

4. Verfahren gemäß Anspruch 3, dadurch gekennzeichnet, daß man den Katalysator in einer Menge einsetzt, die 0,1 bis 0,3 Gew.-% Palladium - bezogen auf 1,3-Propandiol - entspricht.

5. Verfahren gemäß Anspruch 1, dadurch gekennzeichnet, daß man einen Katalysator einsetzt, der zusätzlich Platin und/oder Wismut enthält.

6. Verfahren gemäß Anspruch 5, dadurch gekennzeichnet, daß man einen Katalysator einsetzt, der 4 Gew.-% Palladium, 1 Gew.-% Platin und 5 Gew.-% Wismut enthält.

## Claims

1. A process for the production of an alkali metal salt of 3-hydroxypropionic acid by reaction of propane-1,3-diol with oxygen or an oxygen-containing gas in aqueous alkaline solution in the presence of a palladium-containing supported catalyst, characterized in that
(a) the catalyst is used in a quantity corresponding to 0.1 to 3.0% by weight of palladium, based on propane-1,3-diol and
(b) the reaction is carried out at a temperature of 40 to 55°C.

2. A process as claimed in claim 1, characterized in that the catalyst is used in a quantity corresponding to 0.1 to 1.0% by weight of palladium, based on propane-1,3-diol.

3. A process as claimed in claim 2, characterized in that propane-1,3-diol is used in a quantity corresponding to 6 to 12% by weight, based on the reaction mixture.

4. A process as claimed in claim 3, characterized in that the catalyst is used in a quantity corresponding to 0.1 to 0.3% by weight of palladium, based on propane-1,3-diol.

5. A process as claimed in claim 1, characterized in that a catalyst additionally containing platinum and/or bismuth is used.

6. A process as claimed in claim 5, characterized in that a catalyst containing 4% by weight of palladium, 1% by weight of platinum and 5% by weight of bismuth is used.

## Revendications

1. Procédé de préparation d'un sel de métal alcalin d'acide 3-hydroxypropionique par réaction de 1,3-propandiol avec de l'oxygène ou un gaz contenant de l'oxygène en solution aqueuse alcaline en présence d'un catalyseur sur support contenant du palladium,
caractérisé en ce que,
a) on met en jeu le catalyseur en une quantité, qui correspond à 0,1 jusqu'à 3,0 % en poids de palladium, par rapport au 1,3-propandiol et,
b) on réalise la réaction à une température au niveau de 40 à 55°C.

2. Procédé selon la revendication 1,
caractérisé en ce que,
on met en jeu le catalyseur en une quantité, qui correspond à 0,1 jusqu'à 1 % en poids de palladium, rapporté au 1,3-propandiol.

3. Procédé selon la revendication 2,
caractérisé en ce que,
on utilise du 1,3-propandiol en une quantité, qui correspond de 6 jusqu'à 12 % en poids par rapport au mélange réactionnel.

4. Procédé selon la revendication 3,
caractérisé en ce que,
on utilise le catalyseur en une quantité, qui correspond de 0,1 jusqu'à 0,3 % en poids de palladium, rapporté au 1,3-propandiol.

5. Procédé selon la revendication 1,
caractérisé en ce que,
on utilise un catalyseur, qui contient en supplément du platine et/ou du bismuth.

6. Procédé selon la revendication 5,
caractérisé en ce que,
on utilise un catalyseur, qui contient 4 % en poids de palladium, 1 % en poids de platine et 5 % en poids de bismuth.
